# EUROPEAN PATENT SPECIFICATION

(11) **EP 3 285 881 B1**
(45) Date of publication and mention of the grant of the patent: **12.03.2025**
(21) Application number: 16700199.9
(22) Date of filing: 08.01.2016
(51) Int. Cl.: A61Q 5/02, B65D 83/00, A61K 8/04, A61K 8/73, A45D 34/02, B05B 1/06, B65D 83/20, B65D 83/30, A45D 19/02, B65D 83/14, A45D 34/00

(54) **AEROSOL DEVICE FOR DRY SHAMPOOING AND TREATING THE HAIR**
AEROSOLVORRICHTUNG ZUM TROCKENSCHAMPONIEREN UND BEHANDELN DES HAARES
DISPOSITIF D'AÉROSOL POUR SHAMPOOING SEC ET TRAITEMENT CAPILLAIRE

(30) Priority: 08.01.2015 FR 1550163
(43) Date of publication of application: 28.02.2018
(73) Proprietor: L'OREAL, 75008 Paris (FR)
(72) Inventor: SMAIL, Nadia, 78540 Vernouillet (FR); AUBERT, Lionel, 95270 Asnières Sur Oise (FR); GAWTREY, Jonathan, 92100 Boulogne (FR); ALBISETTI, Nicolas, 95210 Saint Gratien (FR)
(74) Representative: L'Oreal
(86) International application number: PCT/EP2016/050299
(87) International publication number: WO 2016/110578

(56) References cited:
- WO-A2-2016/092108
- FR-A1- 2 985 201
- FR-A1- 2 985 202
- FR-A1- 3 004 929
- US-A- 5 297 739

## Description

The present invention relates to a particular aerosol device comprising a cosmetic composition based on at least one sebum-absorbing powder, and to the use thereof for the dry washing and cosmetic treatment of keratin materials, preferably human keratin fibres such as the hair.

In the field of washing keratin materials, dry shampoos have existed for many years, either in a powder form or in aerosol form. They make it possible to remove excess sebum rapidly without wetting the head of hair. They act by taking up sebum by absorption by means of powders chosen for their sebum-absorbing qualities.

The powders used may be of mineral, organic or synthetic origin and may be wheat, rice and corn starch derivatives.

FR3004929 describes an aerosol device comprising a propellant and a composition comprising a sebum-absorbing powder, the aerosol device comprising a distribution mean with one dispensing orifice, the mean for dispensing the composition does not display a body that is open at its two opposite axial ends, nor an engaging part that is open at its two opposite axial ends.

FR2985201 et FR2985202 discloses a mean for dispensing a cosmetic composition comprising a body that is open at its two opposite axial ends, an engaging part (10) that is open at its two opposite axial ends, at least partially defining a dispensing orifice (12), in particular for application of hair cosmetic composition.

WO2016/092108 discloses a device for dispensing a composition comprising i) a dispensing head intended to be fitted on a container that contains said composition, comprising a body that is open at its two opposite axial ends, an engaging part that is open at its two opposite axial ends, at least partially defining at least one dispensing orifice ii) the composition comprising perlite particles.

In practice, the proposed dry shampoos are not entirely satisfactory, especially in terms of application and comfort. The aerosol sprays conventionally used generate a conical spray. This type of spray is not optimal for a localized application, especially at the root, and is generally uncomfortable to apply due to the relatively large impact force on the head of hair.

There is thus a need to develop a novel aerosol device comprising a dry shampoo composition which offers comfort on application.

There is also a need to develop such a device which offers optimum cleansing activity and gives the head of hair volume.

The applicant has found, surprisingly and advantageously, that the use of a device equipped with a dispensing means comprising a body that is open at its two opposite axial ends and an engaging part that is open at its two opposite axial ends, at least partially defining at least one dispensing orifice, for dispensing a composition comprising a sebum-absorbing powder makes it possible to facilitate the application of the composition with better dispersion and to offer more comfort on application, while at the same time offering the expected cleansing properties of a dry shampoo.

This particular combination allows easy application and uniform, fine and light distribution of the hair composition on the head of hair, thus leading to optimum sebum absorption with a natural result.

The composition according to the invention makes it possible to wash the hair satisfactorily while at the same time especially giving the head of hair suppleness, lightness and softness.

According to a first of its aspects, a subject of the invention is an aerosol device comprising:
- a container containing:
   - one or more propellants, and
   - a composition comprising one or more sebum-absorbing powders with a sebum uptake of greater than or equal to 35 ml/100 g,

   it being possible for the propellant(s) to be present in the composition or, in the container, separate from the composition,
   when the propellant(s) are present in the composition, the sebum absorbent powder(s) are present in an amount ranging from 2% to 15% by weight, relative to the total weight of the composition,
- a means for dispensing said composition comprising:
   - a body that is open at its two opposite axial ends,
   - an engaging part that is open at its two opposite axial ends, at least partially defining at least one dispensing orifice,
   it being understood that:
   - the dispensing orifice is defined between the engaging part and the body;
   - the dispensing orifice has axial symmetry; and
   - the engaging part at least partially defines a plurality of dispensing orifices;
   - the term "sebum uptake" corresponds to the amount of sebum absorbed and/or adsorbed by the powder, expressed in ml of sebum per 100 g of powder and measured using the method for determining the oil uptake of powder described in standard NF T 30-022.

This particular combination makes it possible to offer comfort on application by limiting the impact force of the spray on the head of hair.

It also makes it possible to facilitate the application and distribution of the hair composition homogeneously at the root of the head of hair, and thus makes it possible to afford optimum cleansing.

Moreover, the composition leaves less white residue while at the same time being more styling than the known products.

By combining the sebum-absorbing powder with a styling powder comprising one or more water-insoluble mineral compounds, it is also possible to obtain a styling effect, especially for giving the head of hair volume and mass and for lifting the roots.

According to an other aspect, a subject of the invention is an aerosol device comprising:
- a container containing:
- one or more propellants, and
- a composition comprising one or more sebum-absorbing powders with a sebum uptake of greater than or equal to 35 ml/100 g,
   it being possible for the propellant(s) to be present in the composition or, in the container, separate from the composition,
- a means for dispensing said composition comprising:
- a body (3) that is open at its two opposite axial ends,
- an engaging part (10) that is open at its two opposite axial ends, at least partially defining a dispensing orifice (12),
   providing that said aerosol device is not a device for dispensing a composition, comprising:
   i) a dispensing head (1) intended to be fitted on a container that contains said composition, said device comprising:
      - a body (3) that is open at its two opposite axial ends,
      - an engaging part (10) that is open at its two opposite axial ends, at least partially defining at least one dispensing orifice (12), the cross section of the dispensing orifice being between 0.02 mm² and 0.5 mm²,
   ii) the composition comprising, in particular in a physiologically acceptable medium:
      a) a continuous oily phase, and
      b) at least one deodorant active agent, and
      c) at least one pulverulent base comprising at least:
         1) perlite particles, and
         2) particles of lamellar filler other than perlite, and
         3) optionally at least one fatty acid salt, and
      d) at least one propellant.

The present invention also relates to a process for the dry washing and treatment of the hair, comprising the use of the device as defined previously. In particular, the process comprises a step of spraying using the aerosol device according to the invention, onto keratin materials, of the composition according to the invention.

Other subjects, characteristics, aspects and advantages of the invention will emerge even more clearly on reading the description and the example that follows.

In that which follows and unless otherwise indicated, the limits of a range of values are included within this range, in particular in the expressions "of between" and "ranging from ... to ...".

Moreover, the expression "at least one" used in the present description is equivalent to the expression "one or more".

According to the invention, the aerosol device comprises a container which contains a cosmetic composition comprising one or more sebum-absorbing powders with a sebum uptake of greater than or equal to 35 ml/100 g.

For the purposes of the present invention, the term "sebum-absorbing powder" means a powder that is capable of absorbing and/or adsorbing sebum, which has a sebum uptake of greater than or equal to 35 ml/100 g.

The sebum uptake corresponds to the amount of sebum absorbed and/or adsorbed by the powder. It is expressed in ml of sebum per 100 g of powder and is measured using the method for determining the oil uptake of powder described in standard NF T 30-022.

The oil uptake of powder corresponds to the amount of sebum absorbed onto the available surface of the powder by measuring the "wet point" as indicated below.

The measuring method is as follows: an amount m (in grams) of between 0.5 and 5 g of powder is placed on a glass plate, the amount depending on the density of the powder, followed by dropwise addition of artificial sebum having the following composition:

| | |
|---|---|
| - triolein | 29% by weight |
| - oleic acid | 28.5% by weight |
| - oleyl oleate | 18.5% by weight |
| - squalene | 14% by weight |
| - cholesterol | 7% by weight |
| - cholesteryl palmitate | 3% by weight |

After addition of 4 to 5 drops of artificial sebum, the artificial sebum is incorporated into the powder using a spatula, and addition of the artificial sebum is continued until conglomerates of artificial sebum and powder have formed. From this point, the artificial sebum is added at a rate of one drop at a time and the mixture is subsequently triturated with the spatula.

The addition of artificial sebum is stopped when a firm, smooth paste is obtained. This paste must be able to be spread on the glass plate without cracking or forming lumps. The volume Vs, in ml, of artificial sebum used is then noted.

The sebum uptake corresponds to the ratio Vs/m.

The sebum-absorbing powder(s) used in the aerosol device of the invention have a preferable sebum uptake ranging from 35 to 1000 ml/100 g and better still from 35 to 800 ml/100 g.

Advantageously, the sebum-absorbing particle may have a BET specific surface area of greater than or equal to 150 m²/g, preferably greater than or equal to 300 m²/g, better still greater than 500 m²/g and preferentially greater than 600 m²/g, and especially less than 1500 m²/g.

The BET specific surface area is determined according to the BET (Brunauer-Emmett-Teller) method described in the Journal of the American Chemical Society, vol. 60, page 309, February 1938, which corresponds to International Standard ISO 5794/1 (appendix D). The BET specific surface area corresponds to the total specific surface area (thus including micropores) of the particle and especially of the powder.

The sebum-absorbing powder may be a mineral powder or an organic powder.

More specifically, the sebum-absorbing powder may be chosen from:
starches,
calcium silicates,
perlites,
zeolites,
polylactic acids,
silicas,
polyamide (Nylon) powders,
powders of acrylic polymers, especially of polymethyl methacrylate, of poly(methyl methacrylate/ethylene glycol dimethacrylate), of poly(allyl methacrylate/ethylene glycol dimethacrylate), or of ethylene glycol dimethacrylate/lauryl methacrylate copolymer;
silicone elastomer powders, obtained especially by polymerization of organopolysiloxane containing at least two hydrogen atoms each bonded to a silicon atom and of an organopolysiloxane comprising at least two ethylenically unsaturated groups (especially two vinyl groups) in the presence of a platinum catalyst; and
mixtures thereof.

The sebum-absorbing powder may be a powder coated with a hydrophobic treatment agent.

The hydrophobic treatment agent may be chosen from fatty acids, for instance stearic acid; metal soaps, for instance aluminium dimyristate, the aluminium salt of hydrogenated tallow glutamate; amino acids; N-acylamino acids or salts thereof; lecithin, isopropyl triisostearyl titanate (ITT), and mixtures thereof.

The N-acylamino acids may comprise an acyl group containing from 8 to 22 carbon atoms, for instance a 2-ethylhexanoyl, caproyl, lauroyl, myristoyl, palmitoyl, stearoyl or cocoyl group. The salts of these compounds may be the aluminium, magnesium, calcium, zirconium, zinc, sodium or potassium salts. The amino acid may be, for example, lysine, glutamic acid or alanine.

The term "alkyl" mentioned in the compounds cited above denotes in particular an alkyl group containing from 1 to 30 carbon atoms and preferably containing from 5 to 16 carbon atoms.

The starches that may be used in the present invention are, for example, corn starch, potato starch, tapioca starch, rice starch, wheat starch and cassava starch.

The starches may be modified or unmodified.

A modified starch is a starch that has been modified via processes known to those skilled in the art, for instance esterification, etherification, oxidation, acidic hydrolysis, crosslinking or enzymatic conversion.

Non-limiting examples of modified starches include aluminium starch octenylsuccinate, sodium starch octenylsuccinate, calcium starch octenylsuccinate, distarch phosphate, hydroxyethyl starch phosphate, hydroxypropyl starch phosphate, sodium carboxymethyl starch and sodium starch glycolate.

In a particular embodiment, the starch is a starch octenylsuccinate, in particular of aluminium, the starch being corn, wheat or rice starch. Mention may be made especially of the product sold by Akzo Nobel under the name Dry Flo Plus.

Preferably, the calcium silicates used as sebum-absorbing powder have a sebum uptake of greater than 200 ml/100 g, better still between 400 ml/100 g and 600 ml/100 g and more preferentially of about 475 ml/100 g.

The specific surface area (BET) preferably ranges from about 150 m²/g to 600 m²/g, better still from 300 m²/g to 600 m²/g and even more preferentially from 310 m²/g to 350 m²/g.

The size of the silicate particles is preferably less than 20 micrometres.

These calcium silicates are generally prepared by reaction of reactive silica with an alkaline-earth metal reagent, preferably an alkaline-earth metal oxide or hydroxide, and a source of aluminium such as sodium aluminate or alumina. Since the final properties of the silicate depend on the reactivity of the silica, the preferred source of silica is the product of reaction of a soluble silicate, such as sodium silicate, and of a mineral acid such as sulfuric acid. Suitable amorphous synthetic alkaline-earth metal silicates are manufactured by the company JM Huber Corporation and are sold under the name Hubersorb^{®}. Methods for preparing these silicas are disclosed in greater detail in patent US 4 557 916. Other suitable silicates are available from JM Huber Corporation, for instance the sodium aluminosilicate sold under the brand name Zeolexg and the sodium magnesium aluminosilicate sold under the brand name Hydrex^{®}.

Sebum-absorbing powders that may also be used include perlites, which are generally aluminosilicates of volcanic origin and whose composition is as follows:
70.0-75.0% by weight of silica SiO₂
12.0-15.0% by weight of oxide of aluminium oxide Al₂O₃
3.0-5.0% of sodium oxide Na₂O
3.0-5.0% of potassium oxide K₂O
0.5-2% of iron oxide Fe₂O₃
0.2-0.7% of magnesium oxide MgO
0.5-1.5% of calcium oxide CaO
0.05-0.15% of titanium oxide TiO₂.

Examples of zeolites that may especially be mentioned include sodium or potassium aluminosilicate compounds such as the product sold by Zeochem under the name Xmol.

The polylactic acids that may be used in the present invention are especially Accurel EP600 from Akzo Nobel or the product sold under the name Lactic Acid Polymer 9105 by Dajac Labs.

Silica powders that may be mentioned include:
- the porous silica microspheres sold under the name Silica Beads SB-700 by the company Miyoshi; Sunsphere^{®} H51, Sunsphere^{®} H33 by the company Asahi Glass;
- the polydimethylsiloxane-coated amorphous silica microspheres sold under the name SA Sunsphere^{®} H33 and SA Sunsphere^{®} H53 by the company Asahi Glass.

A Nylon powder that may be mentioned is the Nylon powder sold under the name Orgasol 4000 by the company Atochem.

Acrylic polymer powders that may be mentioned include:
- the polymethyl methacrylate powders sold under the name Covabead^{®} LH85 by the company Wackherr;
- the polymethyl methacrylate/ethylene glycol dimethacrylate powders sold under the name Dow Corning 5640 Microsponge^{®} Skin Oil Adsorber by the company Dow Corning; Ganzpearl^{®} GMP-0820 by the company Ganz Chemical;
- the polyallyl methacrylate/ethylene glycol dimethacrylate powders sold under the name Poly-Pore^{®} L200 or Poly-Pore^{®} E200 by the company Amcol Health and Beauty Solutions Inc.; these powders especially have a sebum uptake of greater than or equal to 1 ml/g, better still ranging from 1 ml/g to 20 ml/g;
- the ethylene glycol dimethacrylate/lauryl methacrylate copolymer powders sold under the name Polytrap^{®} 6603 from the company Dow Corning.

Silicone elastomer powders that may be mentioned include the powders sold under the names Trefil^{®} Powder E-505C and Trefil^{®} Powder E-506C by the company Dow Corning.

Preferably, the sebum-absorbing powder is chosen from modified starches such as starch octenylsuccinates, in particular of aluminium, perlite, polylactic acids and zeolites, and better still from starch octenylsuccinates.

When propellant(s) are present in the composition, the sebum-absorbing powder(s) are present in an amount preferably ranging from 0.1% to 90% by weight, better still from 1% to 30% by weight and even more preferentially from 2% to 15% by weight relative to the total weight of the composition.

The composition may also comprise one or more styling powders comprising one or more water-insoluble inorganic compound(s) different from sebum absorbent powders.

The term "styling powder" is intended to mean a powder constituted of one or more water-insoluble inorganic compound(s) having a capacity for shaping the head of hair or for the durability of this shaping.

The water-insoluble inorganic compound(s) are chosen from metal carbonates, oxides and sulfates and from silicates containing magnesium.

For the purposes of the present invention, the term "water-insoluble" is intended to mean a compound of which the solubility at spontaneous pH in water at 25°C and at atmospheric pressure is less than 0.1%.

Examples include more particularly the carbonates, oxides and sulfates of alkaline earth metals such as beryllium, magnesium, calcium, strontium, barium and radium, better still magnesium and calcium; the oxides, sulfates and carbonates of aluminium, gallium and indium; and silicates containing magnesium, more particularly those containing an amount of magnesium of more than 10% by weight (on a dry basis) as expressed in terms of magnesium oxide, such as Li-Mg-Na silicates, for instance Laponite XLG, which is provided by the company Rockwood.

More preference will be given to using calcium carbonate, magnesium carbonate, alumina, barium sulfate and/or magnesium oxide, and better still calcium carbonate. Preferably, these compounds have a mean particle size of from 20 to 50 µm, as water-insoluble inorganic compound(s).

When they are present, the water-insoluble inorganic compound(s) are present in an amount ranging from 0.1% to 30% by weight, even better still from 0.5% to 15% by weight, and even more preferentially from 1% to 10% by weight, relative to the total weight of the composition, when the propellant(s) are present in the composition.

The composition may also comprise one or more C₂₋C₄ monoalcohols.

C₂₋C₄ monoalcohol(s) which can be used in the aerosol device of the invention include, in particular, ethanol or isopropanol, or better still ethanol.

When they are present, the C₂₋C₄ monoalcohol(s) are preferably present in an amount ranging from 1% to 70% by weight, even better still from 5% to 60% by weight, and even more preferentially from 10% to 50% by weight, relative to the total weight of the composition, when the propellant(s) are present in the composition.

The composition according to the invention may contain one or more additional organic solvents such as polyols, for instance glycerol, propylene glycol or polyethylene glycols.

It may also contain water.

Preferably, the composition according to the invention contains less than 5% by weight of water relative to the total weight of the composition, when the propellant(s) are present in the composition. Even more preferentially, it does not contain any added water. The composition is then said to be anhydrous.

The container of the device according to the invention also comprises one or more propellants.

Examples of propellants that may be used in the aerosol device of the present invention are liquefied gases such as dimethyl ether, 1,1-difluoroethane, or C₃-C₅ alkanes, such as propane, isopropane, n-butane, isobutane or pentane, or compressed gases such as air, nitrogen, carbon dioxide, and mixtures thereof.

Mention may be made preferably of C₃-C₅ alkanes and in particular propane, n-butane, isobutane and mixtures thereof.

The agent(s) may be present in the composition or, as a variant, in the container containing the composition, but separate from the composition.

The agent(s) are preferably present in the composition.

When the propellant(s) are present in the composition, they are preferably present in an amount ranging from 10% to 95% by weight, even better still from 15% to 90% by weight and even more preferentially from 20% to 88% by weight relative to the total weight of the composition.

Preferably, the composition of the invention may also comprise one or more silicones.

Preferably, the additional silicone(s) are silicone gums.

The silicone gums that may be present in the composition according to the invention are especially polydiorganosiloxanes, used alone or as a mixture in a solvent. This solvent can be chosen from volatile silicones, polydimethylsiloxane (PDMS) oils, polyphenylmethylsiloxane (PPMS) oils, isoparaffins, polyisobutylenes, methylene chloride, pentane, dodecane and tridecane, or mixtures thereof.

Examples of silicone gums that may be mentioned more particularly include the following products:
- polydimethylsiloxane gums,
- polydimethylsiloxane-α,ω-disilanol gums,
- polydimethylsiloxane/methylvinylsiloxane gums,
- polydimethylsiloxane/diphenylsiloxane gums,
- polydimethylsiloxane/phenylmethylsiloxane gums,
- polydimethylsiloxane/diphenylsiloxane/methylvinylsiloxane gums.

Products that may be used more particularly are the following mixtures:
- mixtures formed from a polydimethylsiloxane hydroxylated at the end of the chain (known as dimethiconol according to the nomenclature of the CTFA dictionary) and from a linear polydimethylsiloxane (known as dimethicone according to the nomenclature of the CTFA dictionary), such as the product Mirasil D-DML sold by the company Bluestar or the product Xiameter PMX-1503 Fluid sold by the company Dow Corning,
- mixtures formed from a polydimethylsiloxane hydroxylated at the end of the chain (known as dimethiconol according to the nomenclature of the CTFA dictionary) and from a cyclic polydimethylsiloxane (known as cyclomethicone according to the nomenclature of the CTFA dictionary), such as the product Q2 1401 sold by the company Dow Corning.

The high-viscosity silicone gums used in the invention generally have a viscosity of greater than or equal to 0.5 × 10⁻³ m²/s (500 cSt), preferably ranging from 1 × 10⁻³ to 10 × 10⁻³ m²/s. The viscosity is measured using a Brookfield viscometer at 25°C.

When the non-elastomeric silicone(s) are present in the device of the invention, their amount preferably ranges from 0.05% to 5% by weight relative to the total weight of the composition, when the propellant(s) are present in the composition.

The composition of the invention also optionally comprises one or more fatty esters.

The fatty esters optionally used in the invention are liquid or non-liquid.

The term "liquid fatty ester" means an ester derived from a fatty acid and/or from a fatty alcohol that is liquid at standard temperature (25°C) and at atmospheric pressure (760 mmHg, i.e. 1.013 × 10⁵ Pa).

The esters are preferably liquid esters of saturated or unsaturated and linear or branched C₁-C₂₆ aliphatic mono- or polyacids and of saturated or unsaturated and linear or branched C₁-C₂₆ aliphatic mono- or polyalcohols, the total number of carbon atoms in the esters being greater than or equal to 10.

Preferably, for the esters of monoalcohols, at least one from among the alcohol and the acid from which the esters of the invention are derived is branched.

Among the monoesters of monoacids and of monoalcohols, mention may be made of ethyl palmitate, isopropyl palmitate, alkyl myristates, preferably of C₂-C₂₂, such as isopropyl myristate or ethyl myristate, isocetyl stearate, 2-ethylhexyl isononanoate, isononyl isononanoate, isodecyl neopentanoate and isostearyl neopentanoate.

Esters of C₄-C₂₂ dicarboxylic or tricarboxylic acids and of C₁-C₂₂ alcohols and esters of monocarboxylic, dicarboxylic or tricarboxylic acids and of C₄-C₂₆ dihydroxy, trihydroxy, tetrahydroxy or pentahydroxy non-sugar alcohols may also be used.

Mention may be made especially of diethyl sebacate, diisopropyl sebacate, bis(2-ethylhexyl) sebacate, diisopropyl adipate, di-n-propyl adipate, dioctyl adipate, bis(2-ethylhexyl) adipate, diisostearyl adipate, bis(2-ethylhexyl) maleate, triisopropyl citrate, triisocetyl citrate, triisostearyl citrate, glyceryl trilactate, glyceryl trioctanoate, trioctyldodecyl citrate, trioleyl citrate, neopentyl glycol diheptanoate, and diethylene glycol diisononanoate.

The composition may also comprise, as liquid fatty ester, sugar esters and diesters of C₆-C₃₀ and preferably C₁₂-C₂₂ fatty acids. It is recalled that the term "sugar" means oxygen-bearing hydrocarbon-based compounds containing several alcohol functions, with or without aldehyde or ketone functions, and which comprise at least 4 carbon atoms. These sugars may be monosaccharides, oligosaccharides or polysaccharides.

Examples of suitable sugars that may be mentioned include sucrose, glucose, galactose, ribose, fucose, maltose, fructose, mannose, arabinose, xylose and lactose, and derivatives thereof, especially alkyl derivatives, such as methyl derivatives, for instance methylglucose.

The sugar esters of fatty acids may be chosen especially from the group comprising the esters or mixtures of esters of sugars described previously and of linear or branched, saturated or unsaturated C₆-C₃₀ and preferably C₁₂-C₂₂ fatty acids. If they are unsaturated, these compounds may comprise one to three conjugated or unconjugated carbon-carbon double bonds.

The esters according to this variant may also be chosen from mono-, di-, tri- and tetraesters, and polyesters, and mixtures thereof.

These esters may be, for example, oleates, laurates, palmitates, myristates, behenates, cocoates, stearates, linoleates, linolenates, caprates or arachidonates, or mixtures thereof, such as, especially, oleopalmitate, oleostearate or palmitostearate mixed esters.

More particularly, use is made of monoesters and diesters and especially of sucrose, glucose or methylglucose mono- or dioleates, stearates, behenates, oleopalmitates, linoleates, linolenates or oleostearates.

An example that may be mentioned is the product sold under the name Glucate^{®} DO by the company Amerchol, which is a methylglucose dioleate.

Finally, use may also be made of natural or synthetic glycerol esters of mono-, di- or triacids.

Among these, mention may be made of plant oils.

As oils of plant origin or synthetic triglycerides that may be used in the composition of the invention as liquid fatty esters, examples that may be mentioned include:
- triglyceride oils of plant or synthetic origin, such as liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, olive oil, rapeseed oil, coconut oil, wheatgerm oil, sweet almond oil, safflower oil, candlenut oil, camellina oil, tamanu oil, babassu oil and pracaxi oil, caprylic/capric acid triglycerides, for instance those sold by the company Stéarinerie Dubois or those sold under the names Miglyol^{®} 810, 812 and 818 by the company Dynamit Nobel, jojoba oil and shea butter oil.

Fatty esters that will preferably be used include liquid fatty esters derived from monoalcohols, such as isopropyl myristate or palmitate, and more particularly isopropyl myristate.

The non-liquid fatty esters are especially solid esters derived from C₉-C₂₆ fatty acids and from C₉-C₂₆ fatty alcohols.

Among these esters, mention may be made of cetyl lactate, stearyl octanoate, octyl octanoate, cetyl octanoate, decyl oleate, myristyl stearate, octyl palmitate, octyl pelargonate, octyl stearate, alkyl myristates such as cetyl myristate, myristyl myristate or stearyl myristate, and hexyl stearate.

Esters of C₄-C₂₂ dicarboxylic or tricarboxylic acids and of C₁-C₂₂ alcohols and esters of mono-, di- or tricarboxylic acids and of C₂-C₂₆ di-, tri-, tetra- or pentahydroxy alcohols may also be used.

Examples that may especially be mentioned include diethyl sebacate, di-n-propyl adipate, dioctyl adipate and dioctyl maleate.

Among all the esters mentioned above, it is preferred to use myristyl, cetyl or stearyl palmitates, and alkyl myristates such as cetyl myristate, stearyl myristate and myristyl myristate.

When the fatty ester(s) are present in the device of the invention, their amount preferably ranges from 0.1% to 30% by weight and better still from 1% to 10% by weight relative to the total weight of the composition, when the propellant(s) are present in the composition.

The composition contained in the aerosol device of the invention may also comprise propylene carbonate, preferably in an amount ranging from 0.05% to 5% by weight relative to the total weight of the composition, when the propellant(s) are present in the composition.

The compositions defined in the invention may further comprise one or more additives chosen from anionic, cationic, nonionic, amphoteric or zwitterionic conditioning or fixing polymers, fragrances, dyes, protective screening agents, acids, bases, nacres and flakes.

These additives may be present in the composition according to the invention in an amount ranging from 0% to 20% by weight, relative to the total weight of the composition, when the propellant(s) are present in the composition.

Those skilled in the art will take care to choose these optional additives and their amounts so that they do not harm the properties of the compositions of the present invention.

The compositions in accordance with the invention are packaged in an aerosol device comprising a container, also known as a reservoir.

The container is pressurized and comprises the composition to be dispensed. The container containing the pressurized composition may be opaque or transparent. It may be made of glass, of polymer or of metal, optionally coated with a protective varnish coat.

As already mentioned previously, the container contains both the propellant(s) and the other ingredients of the composition, in a single compartment, or as a variant in two compartments. According to the latter variant, the container may be constituted of an outer aerosol can comprising an inner bag hermetically welded to a valve. The various ingredients of the composition are introduced into the inner bag and a propellant is introduced between the bag and the can at a sufficient pressure to make the product come out in the form of a spray.

The container is equipped at its top end with a valve that seals the system.

Onto this valve is fitted a dispensing means, on which the user can press to make the product come out. This dispensing means is also known as a diffuser.

As indicated above, the dispensing means according to the invention comprises a body that is open at its two opposite axial ends and an engaging part that is open at its two opposite axial ends, at least partially defining at least one dispensing orifice.

In particular, the dispensing orifice is preferably defined between the body and the engaging part but may, alternatively, be defined entirely by the engaging part.

By virtue of the device of the invention, a passage is formed through the dispensing means and more particularly through the body and the engaging part, allowing a flow of air to be established through the dispensing means when the product to be dispensed is emitted, and this can prove advantageous when the product is emitted in the form of a spray, allowing a current of air to be created through the dispensing means in order to accompany the flow of the spray.

Moreover, the passage through the dispensing means can be produced with dimensions sufficient to allow, if desired, a finger or a lock of hair to be inserted into this passage. This can make it easier to apply a product to the finger or the lock of hair.

A plurality of dispensing orifices are formed between the body and the engaging part, for example in order to dispense the product in the form of a number of sprays or jets. The number of dispensing orifices can in particular be between, limits included, 2 and 80, preferably between 5 and 60. It may for example be equal to 10. The dispensing orifices each have for example a cross section greater than or equal to 0.0025 mm², better still 0.006 mm² and are preferably spaced apart from one another (measurement along a straight line between the centres of mass of the orifices) by a distance of more than 1 mm.

In another variant, several dispensing orifices are formed entirely in the engaging part. The orifices may be constructed in such a way that the jet exiting from each orifice swirls, especially by virtue of at least two swirl ducts oriented tangentially around the axis of the orifice. The engaging part may have a U-shaped axial half-section. The body may have two concentric mounting skirts between which the engaging part is fastened. The body may comprise a crown into which the engaging part is inserted, the crown possibly bearing one or more reliefs defining, with the engaging part, ducts, especially swirl ducts, for supplying the dispensing orifice.

The body may define a housing that receives the engaging part, which is then called a core.

The dispensing orifice(s) may be open at rest. The expression "at rest" should be understood as meaning before the engaging part is exposed to the pressure of the product to be dispensed. Thus, in this case, the dispensing orifice(s) are already formed and open when the product is sent into the dispensing means in order to be dispensed. Alternatively, the dispensing orifice is formed at the time the product is dispensed, by virtue for example of the elasticity of at least a portion of the body or of the engaging part, which deforms under the pressure of the product at the time it is dispensed.

By virtue of the invention, in the case of spraying, the spray can be emitted at a relatively high flow rate, if desired, while having a dispensing means which has a relatively simple design and functions reliably. In particular, the dispensing orifice may be produced with well-defined dimensions. In addition, the dispensing means may be aesthetically pleasing to the consumer.

The body may have a first surface that flares towards the outside, or converges towards the outside, and the engaging part may have a second surface, opposite the first surface, that diverges towards the outside, or converges towards the outside. The first surface may be conical. The second surface may be conical, with the same angle as the first surface or with a greater or smaller angle.

A different angle that results in a narrowing of the space may lead to an acceleration of the jet before it exits, and this may be advantageous in the context of a spray.

The dispensing orifice(s) may have rotational symmetry, in particular around the dispensing axis. The dispensing axis is defined by the general direction in which the product is dispensed by the dispensing means.

When the dispensing means comprises several dispensing orifices, they preferably have the following characteristics.

Their cross section is advantageously a disc.

They are preferably cylindrical in shape or approximately cylindrical in shape.

The depth of each orifice is advantageously between 0.5 and 2 mm. A long length makes it possible to create an individual spray with a reduced cone so as to create a tubular effect with a sizeable number of orifices. A short length allows a very wide individual spray and even further enlarges the application surface of the multi-orifice diffuser.

The sum of the cross sections of the orifices in the ring is preferably chosen to be close to the surface area of the orifice in the nozzle.

With the same valve, it is possible to obtain various types of spray by choosing the number and the cross section of the orifices. Use may, for example, be made of a dispensing means according to the invention equipped with 80 orifices of 0.005 mm² so as to obtain a gentle mist or a dispensing means according to the invention equipped with 10 orifices of 0.1 mm² so as to obtain a powerful spray.

The orifices may be distributed in various ways. They may be equidistant on the periphery of the ring, equidistant from one another on a portion of the ring, or distributed in equidistant groups composed of several equidistant orifices.

It is possible to create a ring fully supporting the dispensing orifices which may be cylindrical. In this configuration, it is possible to produce small swirl orifices with a different design from the internal and external rings so as to allow the creation of a ring intended to create the "centre post" function at the rear.

The engaging part is preferably attached, thereby making it, and the body, easier to manufacture. Alternatively, the engaging part is moulded in one piece with the body, in particular in the case of the dispensing of a foam, it then being possible for the dispensing orifice to have a larger cross section than in the case of the spraying of a spray.

The space formed between the body and the engaging part is supplied by at least one supply duct, the section of which is preferably greater than that of the dispensing orifice, thereby making it easier to fill this space before the product emerges through the dispensing orifice.

A product dispensing chamber may advantageously be formed, between the engaging part and the body, upstream of the dispensing orifice. This can make the emission of a homogeneous spray, in particular, easier.

The supply duct for the product may open into this chamber, which preferably has an annular shape. Its width, which corresponds to the gap between the engaging part and the body, is preferably greater than the maximum width of the passage, via which the dispensing chamber communicates with the dispensing orifice.

At least one of the body and the engaging part, preferably the body, may have at least one relief for centring the engaging part in relation to the body, and preferably at least ten, better still at least twenty, and even better still at least forty reliefs. These reliefs may extend as far as the edge of the part in which they are produced so as to generate a multitude of orifices via which jets of product exit, the centring reliefs being oriented in particular parallel to the dispensing axis or obliquely in the same circumferential direction around the axis, and optionally also being able to define, between one another, sectional narrowings that cause the jet of product to be accelerated. This or these reliefs are preferably located set back from the dispensing orifice when it is desired to generate a spray in the form of a single jet. The reliefs can be produced on the body, being for example in the form of axial ribs that are distributed regularly around the entire surface of the body opposite the engaging part.

The centring reliefs may optionally ensure alone that the engaging part is held on the body. Alternatively, the engaging part is fixed to the body somewhere other than in the region of the centring reliefs, it being possible in this case for the centring reliefs to have or not have a function of holding the engaging part on the body.

Preferably, the engaging part is fixed in relation to the body. Alternatively, the engaging part is fixed in an adjustable manner in relation to the body, in order for example to allow the user to adjust the width of the dispensing orifice or to close the latter when not in use, for example by screwing it through a quarter turn, this screwing being accompanied by an axial movement of the engaging part in relation to the body.

The engaging part may be flush with the front end of the body so as to generate a spray with an axis substantially parallel to the axis of the engaging part.

The engaging part may extend axially beyond the front end of the body by an amount between 0.01 and 1 mm, better still between 0.01 and 0.5 mm. The spray may then diverge towards the axis of the engaging part.

The engaging part may be axially set back from the front end of the body by an amount between 0.01 and 1 mm, better still between 0.01 and 0.5 mm. The spray may then converge towards the axis of the engaging part.

The invention makes it possible to easily produce, if desired, a dispensing orifice having a circular internal contour. The inside diameter of the passage formed through the dispensing means is for example greater than or equal to 10 mm, better still greater than or equal to 15 mm, 20 mm or 30 mm. When the passage does not have a circular section, the "inside diameter" denotes the diameter of the largest circle inscribed in this passage.

The dispensing means may comprise at least two housings and two engaging parts that are disposed in the housings and each define with the body, at rest, a dispensing orifice according to the invention. The dispensing axes may then be parallel or not parallel, intersecting or not intersecting, for example may converge towards one another.

The dispensing orifice may have, in axial half-section, an axis that converges or diverges in relation to the spraying direction.

The invention also relates to an aerosol device comprising:
- a container containing:
   - one or more propellants, and
   - a composition comprising one or more sebum-absorbing powders with a sebum uptake of greater than or equal to 35 ml/100 g,
   it being possible for the propellant(s) to be present in the composition or, in the container, separate from the composition,
- a means for dispensing said composition comprising:
   a body,
   an engaging part, in particular a core, defining with the body, at rest, at least one dispensing orifice having an annular cross section.

The invention also relates to an aerosol device comprising:
- a container comprising a valve rod or pump rod, containing:
   - one or more propellants, and
   - a composition comprising one or more sebum-absorbing powders with a sebum uptake of greater than or equal to 35 ml/100 g,
   it being possible for the propellant(s) to be present in the composition or, in the container, separate from the composition,
   - a means for dispensing said composition comprising:
      - a body provided with an end piece for connecting to the valve rod or pump rod,
      - a part attached to the body, at least partially defining at least one dispensing orifice having in particular an annular cross section at rest or several dispensing orifices distributed around a dispensing axis (Z),

   the dispensing means not being a through-dispensing means along the dispensing axis (Z),
   the body being closed along the dispensing axis (Z) and said part being in particular of annular shape, or
   the body having a through-opening along the dispensing axis (Z) and said part closing this opening.

The invention may be better understood from reading the following detailed description of non-limiting illustrative embodiments thereof and from examining the appended drawing, in which:
Figure 1 schematically shows a perspective view of an example of a dispensing means produced in accordance with the invention, before the engaging part is fitted on the body of the dispensing means,
Figure 2 shows the dispensing means after the engaging part has been fitted in the body,
Figure 3 is a view similar to Figure 1 in partial section,
Figures 4A to 4F illustrate various arrangements, among others, of the engaging part and the body,
Figure 5 illustrates the possibility of producing the dispensing means with two dispensing orifices according to the invention,
Figure 6 shows a front view of a dispensing means having concentric dispensing orifices,
Figure 7 is an axial section through a variant embodiment of the engaging part,
Figures 8A and 8B are partial front views of different examples of configurations of the engaging part from Figure 7,
Figure 9 is a partial axial section through a variant embodiment of the dispensing orifice,
Figures 10A and 10B are front views along X of different examples of configurations according to Figure 9,
Figure 11 is a view similar to Figure 2 of a variant embodiment of the dispensing means,
Figures 12A to 12C illustrate various examples of arrangements of the reliefs on the body,
Figures 13A to 13C illustrate various examples of configurations of the engaging part with respect to the body,
Figure 14 is a partial axial section through a variant embodiment of the dispensing orifice,
Figure 15 is a section along XV in Figure 14,
Figure 16 is an exemplary embodiment of the body according to Figure 14, and
Figure 17 is a cutaway perspective view of an example of a dispensing means according to the configuration in Figure 14.

In the drawing, the actual respective proportions of the various constituent elements have not always been respected, for the sake of clarity.

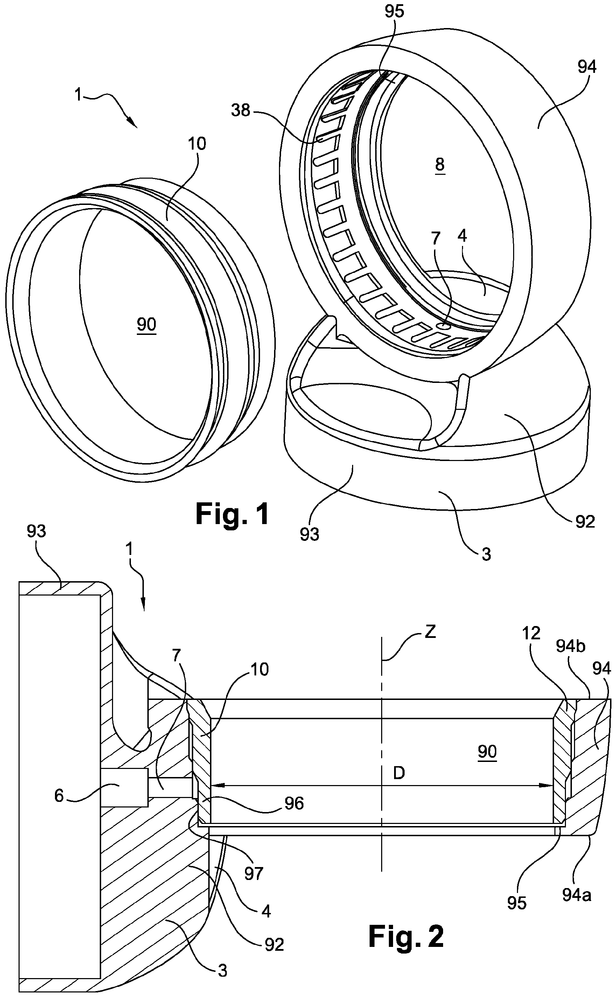

The dispensing means 1 shown in Figures 1 to 3 is intended to be fitted on a container (not shown) provided with a hollow valve rod or hollow pump rod, through which the product to be dispensed that is contained in the container is conveyed towards the dispensing means 1.

The container may in particular be a pressurized container of the aerosol can type, containing a propellant gas such as compressed air, for example, or a liquefied gas.

The container may be provided with a valve and the valve may be opened for example by pressing the hollow rod or alternatively by tilting the latter. When the container is provided with a pump, the pump may be actuated for example by pressing the hollow rod along its longitudinal axis.

The dispensing means 1 comprises a body 3 which may be produced in an integral manner by moulding a single part or may comprise a plurality of elements produced separately and joined together.

The dispensing means 1 may comprise, as can be seen in Figure 2, a housing 6 intended to engage with the hollow rod in order to allow the product delivered through the latter to reach a supply duct 7 which opens into a housing 8 in the body 3. The housing 6 has a size adapted to the outside diameter of the rod, so as to obtain a sealed fit of the rod in the housing 6, in order that the product delivered through the rod passes entirely into the supply duct 7. The latter is for example coaxial with the rod of the container but could be oriented in some other way and have for example a plurality of differently oriented portions.

An engaging part 10, called core in the following text when it is inside the body, is fixed in the housing 8 and defines for example with the body 3 a dispensing orifice 12 having an annular cross section, as illustrated.

The expression "annular cross section" should be understood within the meaning of the present invention as meaning any cross section that follows a closed contour, whether this contour is circular, elliptical, polygonal or some other shape.

Passing axially through the core 10 is an opening 90, the inside diameter D of which may be relatively large, for example greater than or equal to 10 mm, better still 15, 20 or 30 mm.

The opening 90 helps to give the dispensing means a particularly aesthetic appearance. In addition, the opening 90 can allow air to flow through the dispensing means under the entrainment effect of a spray emitted through the dispensing orifice 12. This can help to increase the range of the spray and can increase the freshness effect provided thereby, if need be.

The opening 90 may also allow a finger or a lock of hair to be inserted through the dispensing means, and this can make it possible to apply a product in a single movement over the entire circumference of the element inserted through the dispensing means. This can be an advantage for applying for example an antiseptic or care product to a finger or for treating a lock of hair.

The dispensing axis Z may be perpendicular to the longitudinal axis X of the container on which the dispensing means is fitted, as illustrated.

The dispensing means 1 comprises a base 92 which defines a surface 4 on which the user can press in order to bring about dispensing.

The bottom of the base 92 can be extended by an enclosing skirt 93 which covers the upper part of the container.

The housing 8 which receives the core 10 is defined by a crown 94 of axis Z, the lower side of which is joined to the base 92. The supply duct 7 passes through the base 92 and leads into the housing 8 at a distance from the axial ends, along the axis Z, of the crown 94, being preferably closer to the rear end 94a than to the front end 94b, as can be seen in Figure 2.

The body 3 may have, as illustrated, a shoulder 95 close to the rear end 94a, against which the core 10 can come into axial abutment, if need be, at the end of its fitting.

The core 10 and the housing 8 may have annular surfaces 96 and 97, in sealed contact, in order to close the space formed between the core 10 and the body 3 at the rear of the supply duct 7.

Preferably, the circumferential width I of the dispensing orifice 12, around the spraying direction Z, is constant. If this width I varies, for example so as to take into account the possibly non-uniform pressure drop experienced by the flow of product upstream of the dispensing orifice 12, this does not depart from the scope of the present invention. This non-uniform pressure drop results for example from the geometry of the space between the core and the body, in particular the presence of angles or intersections. By varying the width I, it is possible to ensure that the product can emerge more easily at the point where this pressure drop is greatest, if a spray which is as homogeneous as possible is desired.

The width I of the dispensing orifice is for example between 0.01 and 2 mm.

The core 10 can be fixed to the body 3 in various ways. In the example illustrated in Figures 1 to 3, the core 10 is retained on the body 3 by friction.

In the example illustrated, the core 10 is produced separately from the body 3 and is attached to the latter. The core 10 can be produced from the same thermoplastic material as the body 3 or alternatively from a different thermoplastic material. It is also possible to use a metal material to produce the core 10.

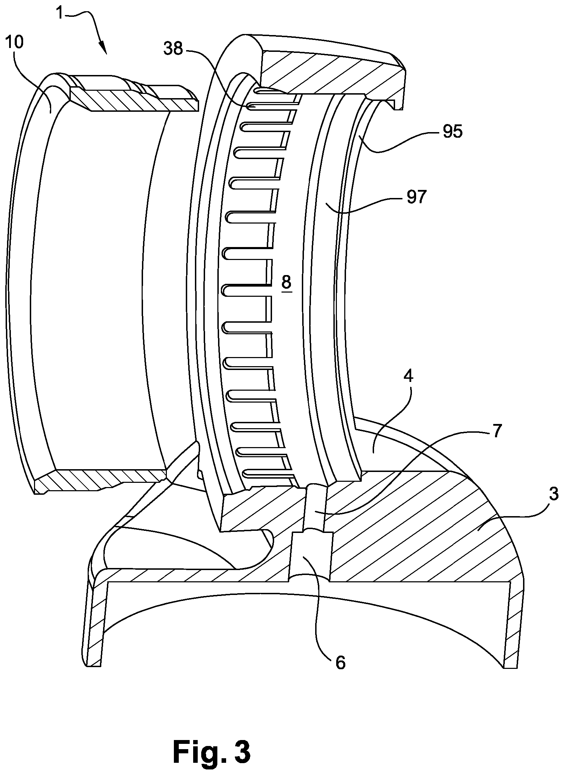

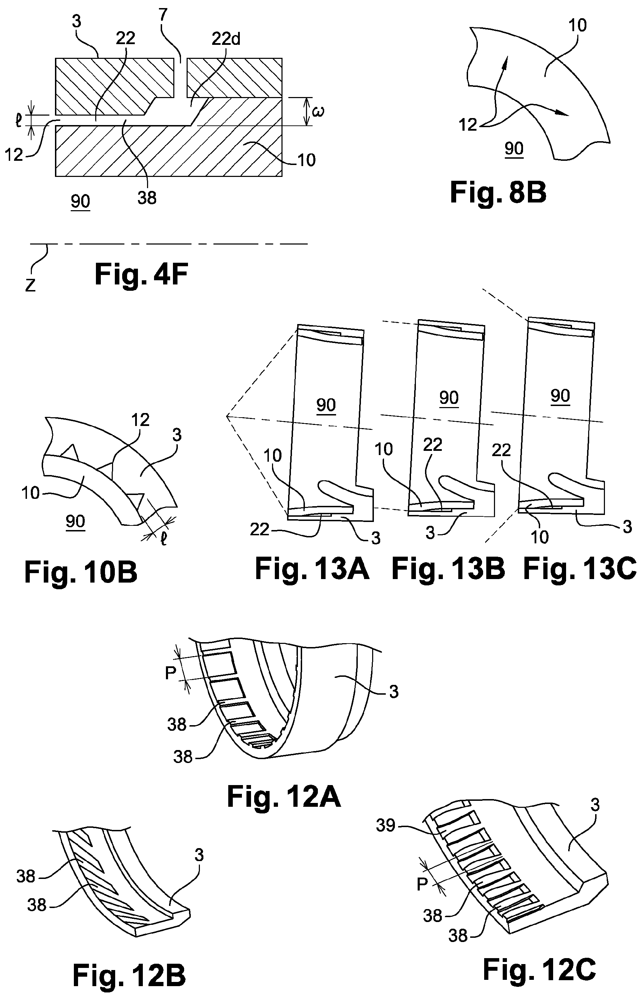

Axial ribs 38 are formed on the internal circumference of the housing 8, as can be seen in particular in Figures 1 and 3, in order to centre the core 10 in the housing 8. The centring reliefs 38 may be, as illustrated in Figures 12A to 12C, parallel or oblique in the circumferential direction with respect to the axis Z, or curved. Each relief 38 may have, when seen in a top view, a contour that is polygonal, in particular rectangular or trapezoidal, or that has a shape that is flared in the direction of the dispensing edge. Two centring reliefs 38 may define, between one another, a narrowing 39 in the vicinity of the dispensing orifice so as to accelerate the fluid via the Venturi effect. The number of centring reliefs 38 is preferably at least 10, better still 20, even better still 40.

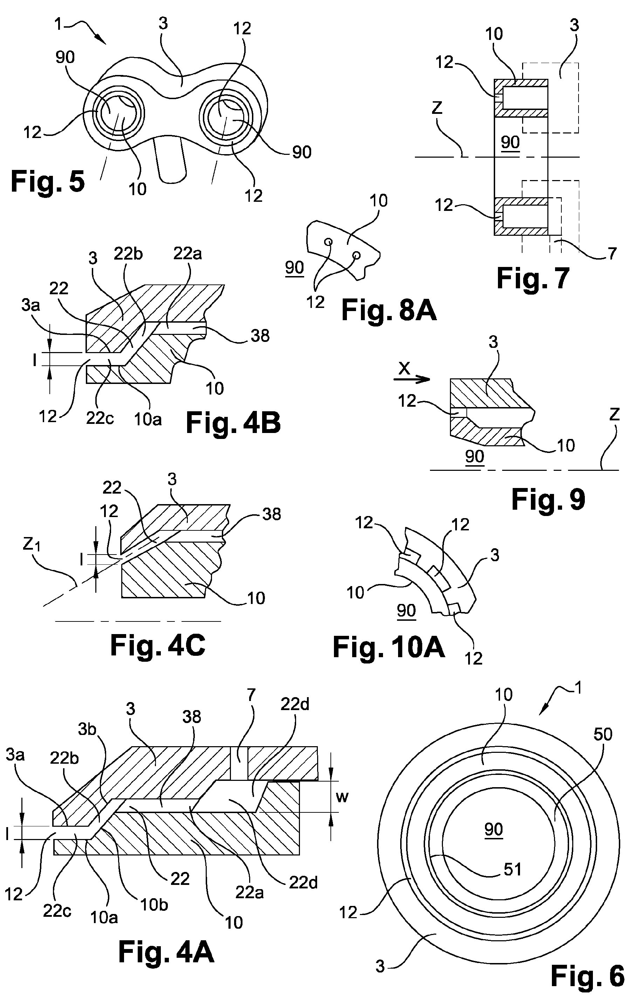

The space 22 formed between the core 10 and the body 3 may have the configuration illustrated schematically in Figure 4A, and open onto the dispensing orifice 12 by way of an annular terminal portion 22c formed between two surfaces 3a and 10a which are in the form of cylinders of revolution about the axis Z.

The terminal wall 22c is attached to a proximal portion 22a by way of an inclined intermediate portion 22b formed between opposite surfaces 3b and 10b.

The centring reliefs 38 extend in the proximal portion 22a. The latter is supplied with product via the dispensing chamber 22d.

When the user actuates the dispensing means 1, the product passes through the supply duct 7 into the space 22 between the core 10 and the body 3 and can be delivered in the form of a spray through the dispensing orifice 12.

In the example in Figures 1 to 3, the spray is continuous angularly around the dispensing axis on account of the absence of contact between the core 10 and the body 3 in the region of the dispensing orifice 12. Specifically, the bearing region or regions between the core 10 and the body 3 are for example located, as illustrated, set back from the dispensing orifice 12 by a distance (measured along the dispensing axis Z) of at least 0.5 mm.

The spray may be discontinuous angularly around the dispensing axis on account of the presence, in particular at the reliefs 38, of contact between the core 10 and the body 3 where the product emerges.

Preferably, the cross section of the supply duct 7 is greater than the section of the dispensing orifice 12 so as to allow the space located upstream of the dispensing orifice to be filled rapidly with the product, this being able to help to form a homogeneous spray right from the start of spraying.

The dispensing chamber 22d formed upstream of the space 22a in which the centring reliefs 38 extend receives the product delivered through the supply duct 7.

The width ω of the dispensing chamber 22d is greater than that I of the terminal portion 22c which opens onto the dispensing orifice 12.

The dispensing chamber 22d improves the dispensing of the product before the latter reaches the narrower portions of the passage through which the product is evacuated.

Figures 4B and 4C illustrate different other examples of possible configurations for the space 22 formed between the core 10 and the body 3 for the product to flow to the dispensing orifice.

In the example in Figure 4B, the space 22 formed between the core and the body comprises a proximal portion 22a in which the reliefs 38 for centring the core 10 in relation to the body 3 extend, extended by an intermediate portion 22b which forms an angle with the spraying direction Z, for example a re-entrant angle. This intermediate portion 22b can be attached to a terminal portion 22c, which opens onto the dispensing orifice 12, this terminal portion being defined for example, as illustrated, between two surfaces 3a and 10a, in the form of cylinders of revolution, parallel to the dispensing direction Z. The variant in Figure 4B does not have a dispensing chamber.

In the variant in Figure 4C, the terminal portion 22c communicates directly with that portion 22a in which the centring reliefs 38 extend. The terminal portion 22c forms, for example, an angle with the dispensing direction Z. Thus, in axial half section, the axis Z1 of the orifice 12 is for example convergent, as illustrated.

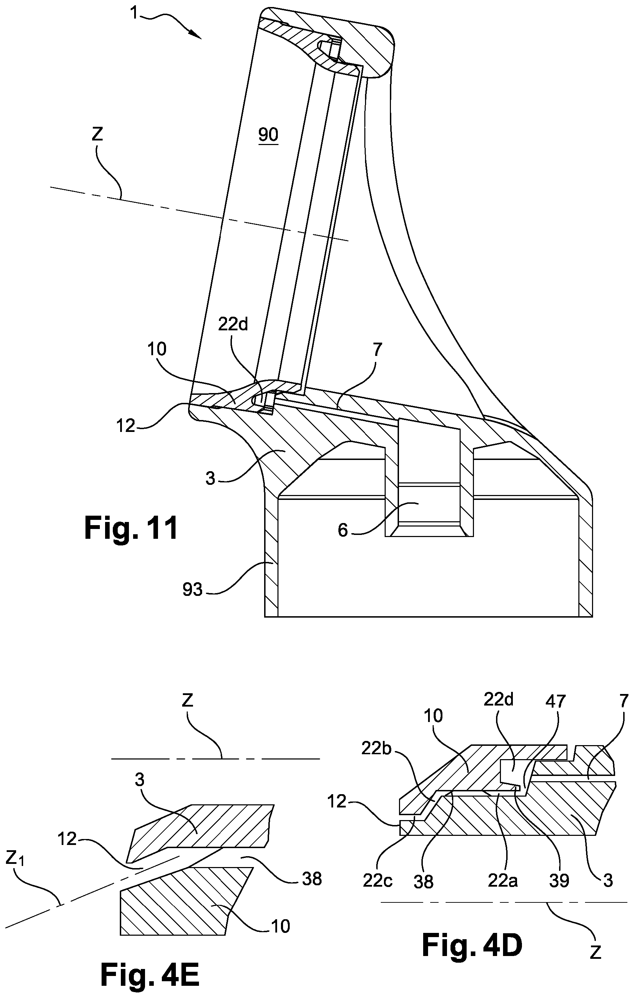

In the variant in figure 4D, the engaging part 10 is outside the body 3. The engaging part 10 is fixed to the body 3 so as to form with the latter the dispensing chamber 22d, facing the supply duct 7. The portions 22a, 22b and 22c allow the product to be conveyed to the dispensing orifice 12.

The supply duct 7 opens for example into the dispensing chamber 22d via a portion oriented parallel to the dispensing axis Z.

Centring reliefs 38 are produced for example on the body 3. The engaging part 10 can be produced, as illustrated, with an annular lip 39 which partially delimits the dispensing chamber 22d and makes it possible to form a narrowing 47 of the section between the chamber 22d and the portion 22a.

Figure 4E illustrates the possibility of having an angle which is divergent between the axis Z2, in axial half-section, of the orifice 12 and the dispensing axis.

In the variant in Figure 4F, the possibility of having no angle between the dispensing axis and the axis Z of the engaging part 10 is illustrated. The supply duct 7 opens for example onto a dispensing chamber 22d. The product is conveyed towards the dispensing orifice 12 via ducts 22 comprising the reliefs 38. The reliefs 38 extend as far as the edge of the dispensing orifice 12 and define a plurality of orifices allowing the product to be delivered in the form of a plurality of jets.

The invention is not limited to a dispensing head comprising only one dispensing orifice 12 produced in accordance with the invention.

By way of example, Figure 5 illustrates a dispensing head 1 which comprises two dispensing orifices 12.

When there are a plurality of dispensing orifices, these may be distributed in multiple ways on the dispensing means. For example, the spraying axes are parallel, or form an angle, in that, for example, they intersect.

Figures 7, 8A and 8B illustrate the possibility for the dispensing means to have a plurality of dispensing orifices 12 formed entirely in the core 10 in order to dispense the product in the form of a plurality of jets for example. The dispensing orifices 12 may have many shapes when observed along their transverse axis, especially being circular or triangular, as illustrated in Figures 8A and 8B. The dispensing orifices 12 may be drilled into the core 10, for example by laser drilling.

The core 10 may have a U-shaped axial half-section, as illustrated in Figure 7. The body 3 may comprise two concentric mounting skirts 41 which define between them a space for mounting the core 10, and may comprise, at its centre, a crown 43 serving to support the engaging part 10. The skirts 41 define, with the crown 43, two annular ducts 45 into which the arms of the U fit. The crown 43 may have, for each orifice 12, two ducts 22 for supplying liquid to this orifice 12.

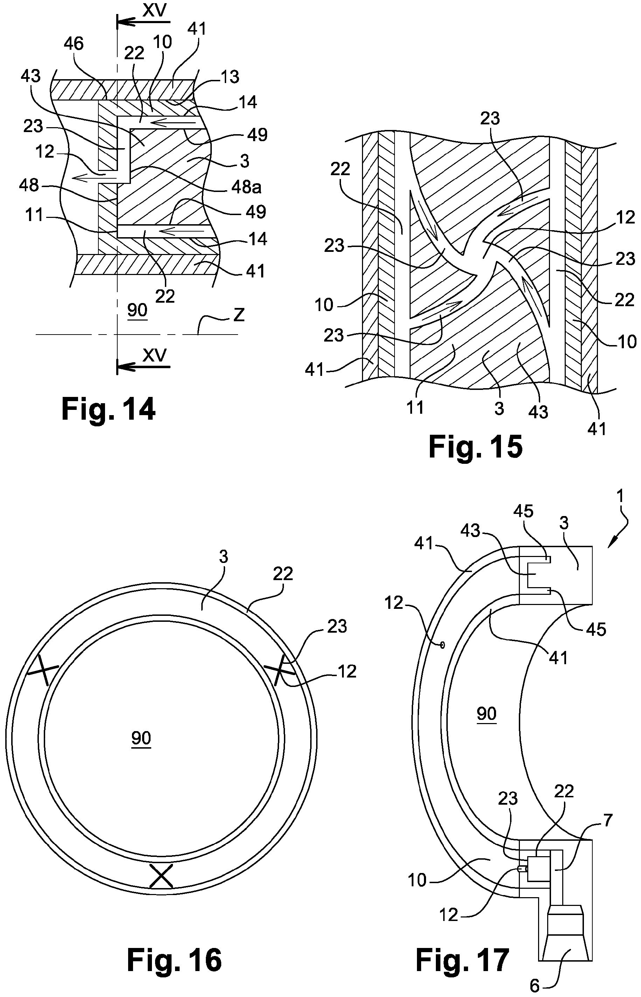

During mounting, as illustrated in Figures 14 and 17, the core 10 may bear against the protrusion 43, the end face 48 of the crown 43 being in contact with the internal face 11 of the core 10. The arms of the U of the core 10 are fixed in the ducts 45, the internal face 46 of the mounting skirts 41 being in contact with the face 13 of the core 10. The internal faces 14 of the arms of the U and the lateral surfaces 49 of the crown 43 may define, between one another, the ducts 22 for supplying liquid to the dispensing orifice 12. The crown 43 may have, especially in the form of impressions, on its outer face 48, supply ducts 23 allowing the liquid to pass from the supply ducts 22 to the dispensing orifice 12.

The core 10 may extend, as illustrated in Figure 13C, forwards relative to the body 3 by an amount between 0 and 1 mm, better still between 0 and 0.5 mm. The spray can then be divergent.

If an additional dispensing orifice is provided, for example by attaching inside the core 10 a second core 50 which defines with the first core 10 a second dispensing orifice 51 which is coaxial with the first dispensing orifice, as illustrated in Figure 6, this does not depart from the scope of the present invention. A passage 90 continues to be formed through the dispensing means.

The dispensing orifice may be supplied with more than one product.

The dispensing means may be supplied with two products which are dispensed through separate dispensing orifices.

It is possible for the axis Z not to be perpendicular to the axis of the rod of the container on which the dispensing means is fitted, as illustrated in Figure 11. In this example, the axis Z is oriented upward when the container is vertical with the dispensing means at the top.

The supply duct 7 can be oriented substantially parallel to the dispensing axis Z, at least in the case of the portion which opens out facing the engaging part 10. The latter may be produced with an annular lip 39 which defines a narrowing of the section 47.

The configuration may be similar to that in Figure 4D apart from the fact that the engaging part 10 is outside the body 3 in the example in Figure 4D and inside it in the example in Figure 11.

The dispensing means may be arranged so as to allow a protective cap to be fitted and to comprise, if need be, an on/off system that makes it possible to prevent the actuation of the device when the dispensing means is in a certain position with respect to the container or when a locking element of the dispensing means is in a certain position in relation to the latter.

In variants which are not illustrated, the dispensing orifice is formed between a body and an engaging part, the body being radially on the inside with respect to the engaging part, the supply duct for the product passing through the body. All of the features described with reference to the figures can be found in variants in which the body is radially on the inside with respect to the engaging part.

The example that follows serves to illustrate the invention.

### EXAMPLE

In the examples that follow, all the amounts are indicated as weight percentage of product as active materials relative to the total weight of the composition.

The following compositions were prepared from the compounds indicated in the table below.

| | **1** | **2** |
|---|---|---|
| Aluminium Starch Octenylsuccinate¹ | 8.6% | 9.39% |
| Calcium carbonate (D50 = 35 µm)² | 2% | 2.18% |
| Hectorite modified with distearyldimethylammonium ide³ | 0.25% | 0.28% |
| Dimethicone/dimethiconol⁴ | 0.55% | - |
| Isopropyl myristate | 2% | 0.42% |
| Propylene carbonate⁵ | 0.07% | - |
| Fragrance | 0.2% | 0.2% |
| Isobutane | 70% | qs 100% |
| Ethanol | qs 100% | - |

| | | |
|---|---|---|
| ¹: Sold under the trade name Dry Flo Plus by National Starch (86% AM) ²: Sold under the trade name Omyacare S60 by Omya ³: Sold under the trade name Bentone 38 by Elementis ⁴: Sold under the trade name Mirasil D-DML by Bluestar ⁵: Sold under the trade name Jeffsol Propylene Carbonate by Huntsman | | |

The aerosol device according to the invention, shown in Figure 1, was used to package the compositions above. It comprises the following characteristics:
- a valve equipped with a nozzle with an orifice 0.64 mm in size and an internal restriction orifice 0.64 mm in size, with an additional gas intake 0.64 mm in size,
- a dispensing means comprising 10 orifices having a unit cross section of 0.25 mm, distributed over the annular surface area.

The compositions were sprayed onto a head of hair. A wide and vaporous diffusion is obtained which allows an extremely fine and light deposit, uniformly distributed over the head of hair.

After drying, it is found that the head of hair is visually cleaner with little visible residue. It is also found that volume is provided.

## Claims

1. Aerosol device comprising:
- a container containing:
- one or more propellants, and
- a composition comprising one or more sebum-absorbing powders with a sebum uptake of greater than or equal to 35 ml/100 g,
it being possible for the propellant(s) to be present in the composition or, in the container, separate from the composition,
when the propellant(s) are present in the composition, the sebum absorbent powder(s) are present in an amount ranging from 2% to 15% by weight, relative to the total weight of the composition,
- a means for dispensing said composition comprising:
- a body (3) that is open at its two opposite axial ends,
- an engaging part (10) that is open at its two opposite axial ends, at least partially defining a dispensing orifice (12),
it being understood that:
- the dispensing orifice (12) is defined between the engaging part (10) and the body (3);
- the dispensing orifice (12) has axial symmetry; and
- the engaging part (10) at least partially defines a plurality of dispensing orifices (12);
- the term "sebum uptake" corresponds to the amount of sebum absorbed and/or adsorbed by the powder, expressed in ml of sebum per 100 g of powder and measured using the method for determining the oil uptake of powder described in standard NF T 30-022.

2. Aerosol device comprising:
- a container containing:
- one or more propellants, and
- a composition comprising one or more sebum-absorbing powders with a sebum uptake of greater than or equal to 35 ml/100 g,
it being possible for the propellant(s) to be present in the composition or, in the container, separate from the composition,
- a means for dispensing said composition comprising:
- a body (3) that is open at its two opposite axial ends,
- an engaging part (10) that is open at its two opposite axial ends, at least partially defining a dispensing orifice (12),
providing that said aerosol device is not a device for dispensing a composition, comprising:
i) a dispensing head (1) intended to be fitted on a container that contains said composition, said device comprising:
- a body (3) that is open at its two opposite axial ends,
- an engaging part (10) that is open at its two opposite axial ends, at least partially defining at least one dispensing orifice (12), the cross section of the dispensing orifice being between 0.02 mm² and 0.5 mm²,
ii) the composition comprising, in particular in a physiologically acceptable medium:
a) a continuous oily phase, and
b) at least one deodorant active agent, and
c) at least one pulverulent base comprising at least:
1) perlite particles, and
2) particles of lamellar filler other than perlite, and
3) optionally at least one fatty acid salt, and
d) at least one propellant.

3. Aerosol device according to Claim 1 or 2, **characterized in that** the sebum-absorbing powder is chosen from modified starches such as starch octenylsuccinates and in particular aluminium starch octenylsuccinate, perlite, polylactic acids and zeolites, and better still from starch octenylsuccinates.

4. Aerosol device according to Claim 2 or 3, **characterized in that**, when the propellant(s) are present in the composition, the sebum absorbent powder(s) are present in an amount ranging from 0.1% to 90% by weight, preferably from 1% to 30% by weight, and even more preferentially from 2% to 15% by weight, relative to the total weight of the composition.

5. Aerosol device according to any one of the preceding claims, **characterized in that** the composition comprises one or more styling powders comprising one or more water-insoluble inorganic compounds different from sebum absorbent powders chosen from metal carbonates, oxides and sulfates and from silicates containing magnesium.

6. Aerosol device according to the preceding claim, **characterized in that** the water-insoluble inorganic compound(s) are chosen from calcium carbonate, magnesium carbonate, alumina, barium sulfate and/or magnesium oxide, and better still calcium carbonate.

7. Aerosol device according to either of Claims 5 and 6, **characterized in that**, when the propellant(s) are present in the composition, the water-insoluble inorganic compound(s) are present in an amount ranging from 0.1% to 30% by weight, even better still from 0.5% to 15% by weight, and even more preferentially from 1% to 10% by weight, relative to the total weight of the composition.

8. Aerosol device according to any one of the preceding claims, **characterized in that** the composition comprises one or more C₂-C₄ monoalcohols, the C₂-C₄ monoalcohol preferably being ethanol.

9. Aerosol device according to Claim 8, **characterized in that**, when the propellant(s) are present in the composition, the C₂-C₄ monoalcohol(s) are present in an amount ranging from 1% to 70% by weight, even better still from 5% to 60% by weight, and even more preferentially from 10% to 50% by weight, relative to the total weight of the composition.

10. Aerosol device according to any one of the preceding claims, **characterized in that** the propellant(s) is or are chosen from air, nitrogen, carbon dioxide, dimethyl ether, C₃-C₅ alkanes, 1,1-difluoroethane and mixtures thereof, preferably chosen from C₃-C₅ alkanes and preferably n-butane, propane, isobutane and mixtures thereof.

11. Aerosol device according to any one of the preceding claims, **characterized in that**, when the propellant(s) are present in the composition, the propellant(s) are present in an amount ranging from 10% to 95% by weight, even better still from 15% to 90% by weight and even more preferentially from 20% to 88% relative to the total weight of the composition.

12. Aerosol device according to any one of the preceding claims, **characterized in that** the composition comprises at least one non-elastomeric silicone and preferably a silicone gum.

13. Aerosol device according to any one of the preceding claims, **characterized in that** the composition comprises at least one silicone gum with a viscosity of greater than 0.5 × 10⁻³ m²/s (500 cSt).

14. Aerosol device according to any one of the preceding claims, **characterized in that** the composition comprises at least one fatty ester, preferably a liquid fatty ester, better still isopropyl myristate.

15. Aerosol device according to any one of the preceding claims, **characterized in that** the composition comprises at least one additive chosen from conditioning or fixing anionic, cationic, nonionic, amphoteric or zwitterionic polymers, fragrances, dyes, protective screening agents, acids, bases, nacres and glitter flakes.

16. Aerosol device according to any one of Claims 1 to 15, **characterized in that** the dispensing orifice (12) has rotational symmetry.

17. Aerosol device according to the preceding claim, **characterized in that** the number of dispensing orifices (12) is between, limits included, 2 and 80, preferably between 5 and 60.

18. Aerosol device according to any one of Claims 1 to 17, **characterized in that** the dispensing orifices (12) each have a cross section greater than or equal to 0.0025 mm².

19. Process for dry-shampooing and treating the hair, which comprises the use of the device according to any one of the preceding claims for applying, to the hair, the composition defined in any one of Claims 1 to 15.

## Patentansprüche

1. Aerosolvorrichtung, umfassend:
- einen Behälter, enthaltend:
- ein oder mehrere Treibmittel und
- eine Zusammensetzung, umfassend ein oder mehrere sebumabsorbierende Pulver mit einer Sebumaufnahme von größer als oder gleich 35 ml/100 g,
wobei das Treibmittel bzw. die Treibmittel in der Zusammensetzung oder in dem Behälter von der Zusammensetzung getrennt vorliegen kann bzw. können,
bei Vorliegen des Treibmittels bzw. der Treibmittel in der Zusammensetzung das sebumabsorbierende Pulver bzw. die sebumabsorbierenden Pulver in einer Menge von 2 bis 15 Gew.-%, bezogen auf das Gesamtgewicht der Zusammensetzung, vorliegt bzw. vorliegen,
- ein Mittel zum Abgeben der Zusammensetzung, umfassend:
- einen Körper (3), der an seinen beiden gegenüberliegenden axialen Enden offen ist,
- einen Eingriffsteil (10), der an seinen beiden gegenüberliegenden axialen Enden offen ist und zumindest teilweise eine Abgabeöffnung (12) definiert,
mit den Maßgaben, dass:
- die Abgabeöffnung (12) zwischen dem Eingriffsteil (10) und dem Körper (3) definiert ist;
- die Abgabeöffnung (12) axiale Symmetrie aufweist; und
- der Eingriffsteil (10) zumindest teilweise mehrere Abgabeöffnungen (12) definiert;
- der Begriff "Sebumaufnahme" der durch das Pulver absorbierten und/oder adsorbierten Sebummenge, ausgedrückt in ml Sebum pro 100 g Pulver und gemessen nach der in der Norm NF T 30-022 beschriebenen Methode zur Bestimmung der Ölaufnahme von Pulver, entspricht.

2. Aerosolvorrichtung, umfassend:
- einen Behälter, enthaltend:
- ein oder mehrere Treibmittel und
- eine Zusammensetzung, umfassend ein oder mehrere sebumabsorbierende Pulver mit einer Sebumaufnahme von größer als oder gleich 35 ml/100 g,
wobei das Treibmittel bzw. die Treibmittel in der Zusammensetzung oder in dem Behälter von der Zusammensetzung getrennt vorliegen kann bzw. können,
- ein Mittel zum Abgeben der Zusammensetzung, umfassend:
- einen Körper (3), der an seinen beiden gegenüberliegenden axialen Enden offen ist,
- einen Eingriffsteil (10), der an seinen beiden gegenüberliegenden axialen Enden offen ist und zumindest teilweise eine Abgabeöffnung (12) definiert,
mit der Maßgabe, dass die Aerosolvorrichtung keine Vorrichtung zum Abgeben einer Zusammensetzung ist, die Folgendes umfasst:
i) einen zum Einbringen an einem Behälter, der die Zusammensetzung enthält, vorgesehenen Abgabekopf (1), wobei die Vorrichtung Folgendes umfasst:
- einen Körper (3), der an seinen beiden gegenüberliegenden axialen Enden offen ist,
- einen Eingriffsteil (10), der an seinen beiden gegenüberliegenden axialen Enden offen ist und zumindest teilweise mindestens eine Abgabeöffnung (12) definiert, wobei der Querschnitt der Abgabeöffnung zwischen 0,02 mm² und 0,5 mm² liegt;
ii) wobei die Zusammensetzung Folgendes umfasst, insbesondere in einem physiologisch unbedenklichen Medium:
a) eine kontinuierliche ölige Phase und
b) mindestens einen Deodorantwirkstoff und
c) mindestens eine pulverförmige Grundlage, die mindestens Folgendes umfasst:
1) Perlitpartikel und
2) Partikel von lamellarem Füllstoff, die von Perlit verschieden sind, und
3) gegebenenfalls mindestens ein Fettsäuresalz, und
d) mindestens ein Treibmittel.

3. Aerosolvorrichtung nach Anspruch 1 oder 2, **dadurch gekennzeichnet, dass** das sebumabsorbierende Pulver aus modifizierten Stärken wie Stärkeoctenylsuccinaten und insbesondere Aluminiumstärkeoctenylsuccinat, Perlit, Polymilchsäuren und Zeolithen und noch besser aus Stärkeoctenylsuccinaten ausgewählt ist.

4. Aerosolvorrichtung nach Anspruch 2 oder 3, **dadurch gekennzeichnet, dass** bei Vorliegen des Treibmittels bzw. der Treibmittel in der Zusammensetzung das sebumabsorbierende Pulver bzw. die sebumabsorbierenden Pulver in einer Menge im Bereich von 0,1 bis 90 Gew.-%, vorzugsweise von 1 bis 30 Gew.-% und noch weiter bevorzugt von 2 bis 15 Gew.-%, bezogen auf das Gesamtgewicht der Zusammensetzung, vorliegt bzw. vorliegen.

5. Aerosolvorrichtung nach einem der vorhergehenden Ansprüche, **dadurch gekennzeichnet, dass** die Zusammensetzung ein oder mehrere Styling-Pulver umfasst, das bzw. die eine oder mehrere wasserunlösliche anorganische Verbindungen, die von sebumabsorbierenden Pulvern verschieden sind, ausgewählt aus Metallcarbonaten, -oxiden und -sulfaten und aus magnesiumhaltigen Silicaten, umfasst bzw. umfassen.

6. Aerosolvorrichtung nach dem vorhergehenden Anspruch, **dadurch gekennzeichnet, dass** die wasserunlösliche anorganische Verbindung bzw. die wasserunlöslichen anorganischen Verbindungen aus Calciumcarbonat, Magnesiumcarbonat, Aluminiumoxid, Bariumsulfat und/oder Magnesiumoxid und noch besser Calciumcarbonat ausgewählt ist bzw. sind.

7. Aerosolvorrichtung nach einem der Ansprüche 5 und 6, **dadurch gekennzeichnet, dass** bei Vorliegen des Treibmittels bzw. der Treibmittel in der Zusammensetzung die wasserunlösliche anorganische Verbindung bzw. die wasserunlöslichen anorganischen Verbindungen in einer Menge im Bereich von 0,1 bis 30 Gew.-%, noch besser von 0,5 bis 15 Gew.-% und noch weiter bevorzugt von 1 bis 10 Gew.-%, bezogen auf das Gesamtgewicht der Zusammensetzung, vorliegt bzw. vorliegen.

8. Aerosolvorrichtung nach einem der vorhergehenden Ansprüche, **dadurch gekennzeichnet, dass** die Zusammensetzung einen oder mehrere C₂-C₄-Monoalkohole umfasst, wobei es sich bei dem C₂-C₄-Monoalkohol vorzugsweise um Ethanol handelt.

9. Aerosolvorrichtung nach Anspruch 8, **dadurch gekennzeichnet, dass** bei Vorliegen des Treibmittels bzw. der Treibmittel in der Zusammensetzung der C₂-C₄-Monoalkohol bzw. die C₂-C₄-Monoalkohole in einer Menge im Bereich von 1 bis 70 Gew.-%, noch besser von 5 bis 60 Gew.-% und noch weiter bevorzugt von 10 bis 50 Gew.-%, bezogen auf das Gesamtgewicht der Zusammensetzung, vorliegt bzw. vorliegen.

10. Aerosolvorrichtung nach einem der vorhergehenden Ansprüche, **dadurch gekennzeichnet, dass** das Treibmittel bzw. die Treibmittel aus Luft, Stickstoff, Kohlendioxid, Dimethylether, C₃-C₅-Alkanen, 1,1-Difluorethan und Mischungen davon, vorzugsweise aus C₃-C₅-Alkanen und vorzugsweise n-Butan, Propan, Isobutan und Mischungen davon ausgewählt ist bzw. sind.

11. Aerosolvorrichtung nach einem der vorhergehenden Ansprüche, **dadurch gekennzeichnet, dass** bei Vorliegen des Treibmittels bzw. der Treibmittel in der Zusammensetzung das Treibmittel bzw. die Treibmittel in einer Menge im Bereich von 10 bis 95 Gew.-%, noch besser von 15 bis 90 Gew.-% und noch weiter bevorzugt von 20 bis 88 Gew.-%, bezogen auf das Gesamtgewicht der Zusammensetzung, vorliegt bzw. vorliegen.

12. Aerosolvorrichtung nach einem der vorhergehenden Ansprüche, **dadurch gekennzeichnet, dass** die Zusammensetzung mindestens ein nichtelastomeres Silikon und vorzugsweise ein Silikongummi umfasst.

13. Aerosolvorrichtung nach einem der vorhergehenden Ansprüche, **dadurch gekennzeichnet, dass** die Zusammensetzung mindestens ein Silikongummi mit einer Viskosität von mehr als 0,5 × 10⁻³ m²/s (500 cSt) umfasst.

14. Aerosolvorrichtung nach einem der vorhergehenden Ansprüche, **dadurch gekennzeichnet, dass** die Zusammensetzung mindestens einen Fettsäureester, vorzugsweise einen flüssigen Fettsäureester, noch besser Isopropylmyristat, umfasst.

15. Aerosolvorrichtung nach einem der vorhergehenden Ansprüche, **dadurch gekennzeichnet, dass** die Zusammensetzung mindestens ein Additiv umfasst, das aus konditionierenden oder fixierenden anionischen, kationischen, nichtionischen, amphoteren oder zwitterionischen Polymeren, Duftstoffen, Farbstoffen, Schutzfiltern, Säuren, Basen, Perlglanzstoffen und Glitzerflocken ausgewählt ist.

16. Aerosolvorrichtung nach einem der Ansprüche 1 bis 15, **dadurch gekennzeichnet, dass** die Abgabeöffnung (12) Rotationssymmetrie aufweist.

17. Aerosolvorrichtung nach dem vorhergehenden Anspruch, **dadurch gekennzeichnet, dass** die Zahl der Abgabeöffnungen (12) zwischen 2 und 80, vorzugsweise zwischen 5 und 60, Grenzen eingeschlossen, liegt.

18. Aerosolvorrichtung nach einem der Ansprüche 1 bis 17, **dadurch gekennzeichnet, dass** die Abgabeöffnungen (12) jeweils einen Querschnitt größer oder gleich 0,0025 mm² aufweisen.

19. Verfahren zum Trockenshampoonieren und Behandeln des Haars, das die Verwendung der Vorrichtung nach einem der vorhergehenden Ansprüche zum Aufbringen der in einem der Ansprüche 1 bis 15 definierten Zusammensetzung auf das Haar umfasst.

## Revendications

1. Dispositif aérosol comprenant :
- un récipient contenant :
- un ou plusieurs agents propulseurs, et
- une composition comprenant une ou plusieurs poudres absorbantes de sébum dont une absorption de sébum est supérieure ou égale à 35 ml/100 g,
l'agent ou les agents propulseurs pouvant être présents dans la composition ou, dans le récipient, séparés de la composition,
lorsque l'agent ou les agents propulseurs sont présents dans la composition, la ou les poudres absorbantes de sébum sont présentes en une quantité allant de 2 % à 15 % en poids, par rapport au poids total de la composition,
- un moyen de distribution de ladite composition comprenant :
- un corps (3) ouvert au niveau de ses deux extrémités axiales opposées,
- une partie d'engagement (10) ouverte au niveau de ses deux extrémités axiales opposées, définissant au moins partiellement un orifice de distribution (12),
étant entendu que :
- l'orifice de distribution (12) est défini entre la partie d'engagement (10) et le corps (3) ;
- l'orifice de distribution (12) a une symétrie axiale ; et
- la partie d'engagement (10) définit au moins partiellement une pluralité d'orifices de distribution (12) ;
- les termes « absorption de sébum » correspondent à la quantité de sébum absorbée et/ou adsorbée par la poudre, exprimée en ml de sébum pour 100 g de poudre et mesurée selon le procédé de détermination de l'absorption d'huile par une poudre décrite dans la norme NF T 30-022.

2. Dispositif aérosol comprenant :
- un récipient contenant :
- un ou plusieurs agents propulseurs, et
- une composition comprenant une ou plusieurs poudres absorbantes de sébum dont une absorption de sébum est supérieure ou égale à 35 ml/100 g,
l'agent ou les agents propulseurs pouvant être présents dans la composition ou, dans le récipient, séparés de la composition,
- un moyen de distribution de ladite composition comprenant :
- un corps (3) ouvert au niveau de ses deux extrémités axiales opposées,
- une partie d'engagement (10) ouverte au niveau de ses deux extrémités axiales opposées, définissant au moins partiellement un orifice de distribution (12),
à condition que ledit dispositif aérosol ne soit pas un dispositif de distribution d'une composition, comprenant :
i) une tête de distribution (1) destinée à être montée sur un récipient qui contient ladite composition, ledit dispositif comprenant :
- un corps (3) ouvert au niveau de ses deux extrémités axiales opposées,
- une partie d'engagement (10) ouverte au niveau de ses deux extrémités axiales opposées, définissant au moins partiellement au moins un orifice de distribution (12), la section transversale de l'orifice de distribution étant comprise entre 0,02 mm² et 0,5 mm²,
ii) la composition comprenant, en particulier dans un milieu physiologiquement acceptable :
a) une phase huileuse continue, et
b) au moins un agent actif déodorant, et
c) au moins une base pulvérulente comprenant au moins :
1) des particules de perlite, et
2) des particules de charge lamellaire autres que la perlite, et
3) éventuellement au moins un sel d'acide gras, et
d) au moins un agent propulseur.

3. Dispositif aérosol selon la revendication 1 ou 2, **caractérisé en ce que** la poudre absorbante de sébum est choisie parmi les amidons modifiés tels que les octénylsuccinates d'amidon et en particulier l'octénylsuccinate d'amidon d'aluminium, la perlite, les acides polylactiques et les zéolithes, et mieux parmi les octénylsuccinates d'amidon.

4. Dispositif aérosol selon la revendication 2 ou 3, **caractérisé en ce que**, lorsque l'agent ou les agents propulseurs sont présents dans la composition, la ou les poudres absorbantes de sébum sont présentes en une quantité allant de 0,1 % à 90 % en poids, de préférence de 1 % à 30 % en poids, et encore plus préférentiellement de 2 % à 15 % en poids, par rapport au poids total de la composition.

5. Dispositif aérosol selon l'une quelconque des revendications précédentes, **caractérisé en ce que** la composition comprend une ou plusieurs poudres coiffantes comprenant un ou plusieurs composés inorganiques insolubles dans l'eau différents des poudres absorbantes de sébum choisis parmi les carbonates, oxydes et sulfates métalliques et parmi les silicates contenant du magnésium.

6. Dispositif aérosol selon la revendication précédente, **caractérisé en ce que** le ou les composés inorganiques insolubles dans l'eau sont choisis parmi le carbonate de calcium, le carbonate de magnésium, l'alumine, le sulfate de baryum et/ou l'oxyde de magnésium, et mieux le carbonate de calcium.

7. Dispositif aérosol selon l'une quelconque des revendications 5 ou 6, **caractérisé en ce que**, lorsque l'agent ou les agents propulseurs sont présents dans la composition, le ou les composés inorganiques insolubles dans l'eau sont présents en une quantité allant de 0,1 % à 30 % en poids, encore mieux de 0,5 % à 15 % en poids, et encore plus préférentiellement de 1 % à 10 % en poids, par rapport au poids total de la composition.

8. Dispositif aérosol selon l'une quelconque des revendications précédentes, **caractérisé en ce que** la composition comprend un ou plusieurs monoalcools en C₂-C₄, le monoalcool en C₂-C₄ étant de préférence l'éthanol.

9. Dispositif aérosol selon la revendication 8, **caractérisé en ce que**, lorsque l'agent ou les agents propulseurs sont présents dans la composition, le ou les monoalcools en C₂-C₄ sont présents en une quantité allant de 1 % à 70 % en poids, encore mieux de 5 % à 60 % en poids, et encore plus préférentiellement de 10 % à 50 % en poids, par rapport au poids total de la composition.

10. Dispositif aérosol selon l'une quelconque des revendications précédentes, **caractérisé en ce que** l'agent ou les agents propulseurs sont choisis parmi l'air, l'azote, le dioxyde de carbone, le diméthyléther, les alcanes en C₃-C₅, le 1,1-difluoroéthane et leurs mélanges, de préférence choisis parmi les alcanes en C₃-C₅ et de préférence le n-butane, le propane, l'isobutane et leurs mélanges.

11. Dispositif aérosol selon l'une quelconque des revendications précédentes, **caractérisé en ce que**, lorsque l'agent ou les agents propulseurs sont présents dans la composition, l'agent ou les agents propulseurs sont présents en une quantité allant de 10 % à 95 % en poids, encore mieux de 15 % à 90 % en poids et encore plus préférentiellement de 20 % à 88 % par rapport au poids total de la composition.

12. Dispositif aérosol selon l'une quelconque des revendications précédentes, **caractérisé en ce que** la composition comprend au moins une silicone non élastomérique et de préférence une gomme de silicone.

13. Dispositif aérosol selon l'une quelconque des revendications précédentes, **caractérisé en ce que** la composition comprend au moins une gomme de silicone ayant une viscosité supérieure à 0,5 × 10⁻³ m²/s (500 cSt).

14. Dispositif aérosol selon l'une quelconque des revendications précédentes, **caractérisé en ce que** la composition comprend au moins un ester gras, de préférence un ester gras liquide, mieux le myristate d'isopropyle.

15. Dispositif aérosol selon l'une quelconque des revendications précédentes, **caractérisé en ce que** la composition comprend au moins un additif choisi parmi les polymères anioniques, cationiques, non ioniques, amphotères ou zwittérioniques, conditionneurs ou fixateurs, les fragrances, les colorants, les filtres protecteurs, les acides, les bases, les nacres et les paillettes scintillantes.

16. Dispositif aérosol selon l'une quelconque des revendications 1 à 15, **caractérisé en ce que** l'orifice de distribution (12) présente une symétrie de révolution.

17. Dispositif aérosol selon la revendication précédente, **caractérisé en ce que** le nombre d'orifices de distribution (12) est compris entre, limites comprises, 2 et 80, de préférence entre 5 et 60.

18. Dispositif aérosol selon l'une quelconque des revendications 1 à 17, **caractérisé en ce que** les orifices de distribution (12) ont chacun une section transversale supérieure ou égale à 0,0025 mm².

19. Procédé pour le shampooinage à sec et le traitement des cheveux, qui comprend l'utilisation du dispositif selon l'une quelconque des revendications précédentes pour appliquer, sur les cheveux, la composition définie dans l'une quelconque des revendications 1 à 15.
